# EUROPÄISCHE PATENTSCHRIFT

(11) **EP 4 377 290 B1**
(45) Veröffentlichungstag und Bekanntmachung des Hinweises auf die Patenterteilung: **09.07.2025**
(21) Anmeldenummer: 22751382.7
(22) Anmeldetag: 19.07.2022
(51) Int. Cl.: C07C 57/05, C07C 57/055, C07C 57/07

(54) **VERFAHREN ZUR HERSTELLUNG VON ACRYLSÄURE**
PROCESS FOR PREPARING ACRYLIC ACID
PROCÉDÉ DE PRODUCTION D'ACIDE ACRYLIQUE

(30) Priorität: 28.07.2021 EP 21188252
(43) Veröffentlichungstag der Anmeldung: 05.06.2024
(73) Patentinhaber: BASF SE, 67056 Ludwigshafen am Rhein (DE)
(72) Erfinder: GIESHOFF, Tile, 67056 Ludwigshafen (DE); HAMMON, Ulrich, 68163 Mannheim (DE); SCHROEDER, Juergen, 67056 Ludwigshafen (DE); ZUROWSKI, Peter, 67056 Ludwigshafen (DE); REIN, Christian, 67056 Ludwigshafen (DE); CRAUWELS, Filip, 2040 Antwerpen (BE)
(74) Vertreter: BASF IP Association
(86) Internationale Anmeldenummer: PCT/EP2022/070166
(87) Internationale Veröffentlichungsnummer: WO 2023/006503

(56) Entgegenhaltungen:
- EP-A1- 2 114 852
- EP-B1- 2 114 852

## Beschreibung

Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt, das Produktgasgemisch anschließend in eine mit trennwirksamen Einbauten ausgerüstete Kondensationskolonne leitet, das Produktgasgemisch innerhalb der Kondensationskolonne in sich selbst aufsteigen lässt und dabei fraktionierend kondensiert, wobei das Produktgasgemisch in eine schwerer als Acrylsäure siedende Folgeprodukte und Nebenkomponenten enthaltende Sumpfflüssigkeit, eine Wasser und Nebenkomponenten insgesamt entreichert enthaltende rohe Acrylsäure als Zielprodukt, ein noch Acrylsäure und Nebenkomponenten enthaltendes saures Wasser und ein tiefer als Wasser siedende Nebenkomponenten enthaltendes Restgasgemisch aufgetrennt wird, das Zielprodukt über einen Seitenabzug aus der Kondensationskolonne herausführt und sich der Seitenabzug oberhalb der Zuführstelle des Produktgasgemischs in die Kondensationskolonne befindet, wobei die produktberührten Teile der Kondensationskolonne aus nichtrostendem Stahl sind, mindestens einer der Kondensationskolonne zugeführten Stoffströme eine Quelle für Halogenid-Ionen enthält und im Bereich der trennwirksamen Einbauten der Kondensationskolonne oberhalb des Seitenabzugs Halogenid-Ionen entfernt werden.

Acrylsäure ist ein bedeutendes Zwischenprodukt, das beispielsweise im Rahmen der Herstellung von Polymerdispersionen (gegebenenfalls auch in Form ihrer Ester mit Alkanolen) sowie von Wasser superabsorbierenden Polymeren Verwendung findet.

Acrylsäure ist unter anderem durch heterogen katalysierte Gasphasen-Partialoxidation von C₃-Vorläufern (von C₃-Vorläuferverbindungen) der Acrylsäure (unter diesem Begriff sollen insbesondere solche chemischen Verbindungen zusammengefasst werden, die formal durch Reduktion von Acrylsäure erhältlich sind; bekannte C₃-Vorläufer von Acrylsäure sind z.B. Propan, Propen, Acrolein, Propionaldehyd und Propionsäure; der Begriff soll aber auch Vorläuferverbindungen der vorgenannten Verbindungen umfassen, wie z.B. Glycerin (von Glycerin ausgehend kann Acrylsäure beispielsweise durch heterogen katalysierte oxidative Dehydratisierung in der Gasphase erzeugt werden; vgl. z. B. EP 1 710 227 A1, WO 06/114506 und WO 06/092272) mit molekularem Sauerstoff an im festen Aggregatzustand befindlichen Katalysatoren bei erhöhter Temperatur erhältlich.

Dabei werden die genannten Ausgangsgase, in der Regel mit inerten Gasen wie z. B. Stickstoff, CO₂, gesättigten Kohlenwasserstoffen und/oder Wasserdampf verdünnt, im Gemisch mit molekularem Sauerstoff bei erhöhten Temperaturen sowie gegebenenfalls erhöhtem Druck über (z.B. übergangsmetallische) Mischoxidkatalysatoren geleitet und oxidativ in ein Acrylsäure, Wasser sowie unerwünschten Nebenprodukte wie z.B. Furfurale, Benzaldehyd, Aceton, Formaldehyd und Maleinsäureanhydrid etc. enthaltendes Produktgasgemisch umgewandelt, aus welchem die Acrylsäure abgetrennt werden muss (die Nebenprodukte und die von Wasserdampf verschiedenen inerten Verdünnungsgase sollen in dieser Schrift unter dem Begriff "Nebenkomponenten" zusammengefasst werden; außerdem soll dieser Begriff die bei den Acrylsäureabtrennverfahren üblicherweise zugesetzten Polymerisationsinhibitoren umfassen).

Aus den Schriften DE 199 24 533 A1, DE 199 24 532 A1, WO 01/77056, DE 101 56 016 A1, DE 102 43 625 A1, DE 102 23 058 A1, DE 102 35 847 A1, WO 2004/035514, WO 00/53560, DE 103 32 758 A1 und EP 2 114 852 A1 sind wie eingangs beschriebene Verfahren zur Herstellung von Acrylsäure bekannt, bei denen eine Grundabtrennung einer rohen Acrylsäure durch fraktionierende Kondensation des Produktgasgemisches der heterogen katalysierten Gasphasen-Partialoxidation vorgenommen wird. Der Begriff rohe Acrylsäure bzw. Rohacrylsäure bringt dabei zum Ausdruck, dass es sich bei der über den ersten Seitenabzug entnommenen Acrylsäure um kein reines Produkt, sondern um ein Gemisch handelt, das neben Acrylsäure (in der Regel ≥50 oder ≥60 Gew.-%, meist ≥70 oder ≥80 Gew.-%, vielfach ≥90 Gew.-% und häufig ≥95 Gew.-% oder mehr des Gesamtgewichtes) noch Wasser und Nebenkomponenten wie z.B. niedere Aldehyde (z.B. Furfurale, Acrolein, Benzaldehyd), niedere Carbonsäuren (z.B. Essigsäure, Propionsäure, Ameisensäure) etc. enthält. In jedem Fall ist der Gesamtgehalt an Wasser und Nebenkomponenten, bezogen auf den Gehalt an Acrylsäure, in der rohen Acrylsäure geringer als im Produktgasgemisch der Gasphasen-Partialoxidation, weshalb man auch sagt, dass die rohe Acrylsäure diese Bestandteile insgesamt abgereichert enthält (einzelne Bestandteile können hingegen in der rohen Acrylsäure vergleichsweise angereichert enthalten sein).

Teilweise ist die Reinheit der so abgetrennten rohen Acrylsäure bereits für den ins Auge gefassten Verwendungszweck der Acrylsäure ausreichend (z.B. zum Zweck der Veresterung derselben, oder zum Zweck des Aufbaus von durch radikalische Polymerisation erhältlichen Polymeren). Vielfach wird man die abgetrennte rohe Acrylsäure jedoch wenigstens einem weiteren thermischen Trennverfahren unterwerfen, um aus der rohen Acrylsäure eine reinere (eine einen im Vergleich zur rohen Acrylsäure höheren Acrylsäuregehalt in Gew.-% aufweisende) Acrylsäure zu gewinnen, die den für den jeweiligen Verwendungszweck erforderlichen Reinheitsgrad aufweist.

Unter thermischen Trennverfahren werden dabei solche verstanden, bei denen unter Zufuhr oder unter Entzug von (in der Regel thermischer) Energie ein physikalisch wenigstens zweiphasiges System erzeugt wird, wobei es infolge der zwischen den Phasen bestehenden Temperatur- und Stoffmengengradienten zu einem Wärme- und Stoffaustausch kommt, der letztlich die gewünschte Auftrennung bedingt, und die Extraktion.

Häufig werden thermische Trennverfahren in trennwirksame Einbauten enthaltenden Trennkolonnen durchgeführt, in denen die vorgenannten wenigstens zwei stofflichen Phasen in der Regel zueinander im Gegenstrom geführt werden. Vielfach ist eine der beiden stofflichen Phasen gasförmig (sie wird in einer Trennkolonne in der Regel als aufsteigende Phase geführt) und die andere flüssig (sie wird in einer Trennkolonne in der Regel als absteigende Phase geführt). Grundsätzlich können die wenigstens zwei stofflichen Phasen aber auch flüssig (z.B. im Fall einer Extraktion) oder fest und flüssig (z.B. im Fall einer Kristallisation), oder fest und gasförmig (z.B. im Fall einer Adsorption) sein.

Beispiele für Fallgestaltungen thermischer Trennverfahren, bei denen eine der wenigstens zwei stofflichen Phasen flüssig und eine gasförmig ist, und damit natürliches Element des in dieser Schrift verwendeten Begriffs "thermische Trennverfahren", sind die Rektifikation (eine aufsteigende Dampfphase wird in der Trennkolonne im Gegenstrom zu einer absteigenden Flüssigphase geführt), und die Desorption (der Umkehrprozess zur Absorption; das in einer Flüssigphase gelöste Gas wird durch Erniedrigung des Drucks über der Flüssigphase, durch Erhöhung der Temperatur der Flüssigphase und/oder durch Hindurchführen einer Gasphase durch die Flüssigphase aus der Flüssigphase herausgeführt; ist die Hindurchführung einer Gasphase beteiligt, wird die Desorption auch als Strippung bezeichnet). Aber auch die Absorption (in der Regel wird ein in einer Trennkolonne aufsteigendes Gas zu wenigstens einem in der Trennkolonne flüssig absteigenden Absorptionsmittel im Gegenstrom geführt) und die fraktionierende Kondensation eines Gasgemischs (Gas-/Flüssigphase Beispiel) sind Bestandteil des Begriffs thermisches Trennverfahren. Ein besonders günstiges thermisches Trennverfahren zur Weiterreinigung von roher Acrylsäure ist die kristallisative Weiterreinigung (die Kristallisation).

Bei der fraktionierenden Kondensation des Produktgasgemisches der heterogen katalysierten Gasphasen-Partialoxidation wird in der verwendeten Kondensationskolonne gelegentlich und unerwartet Korrosion festgestellt.

Es bestand die Aufgabe diese unerwartete Korrosion zu verhindern.

Die Aufgabe wird gelöst durch ein Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt, das Produktgasgemisch anschließend in eine mit trennwirksamen Einbauten ausgerüstete Kondensationskolonne leitet, das Produktgasgemisch innerhalb der Kondensationskolonne in sich selbst aufsteigen lässt und dabei fraktionierend kondensiert, wobei das Produktgasgemisch in eine schwerer als Acrylsäure siedende Folgeprodukte und Nebenkomponenten enthaltende Sumpfflüssigkeit, eine Wasser und Nebenkomponenten insgesamt entreichert enthaltende rohe Acrylsäure als Zielprodukt, ein noch Acrylsäure und Nebenkomponenten enthaltendes saures Wasser und ein tiefer als Wasser siedende Nebenkomponenten enthaltendes Restgasgemisch aufgetrennt wird, das Zielprodukt über einen Seitenabzug aus der Kondensationskolonne herausführt und sich der Seitenabzug oberhalb der Zuführstelle des Produktgasgemischs in die Kondensationskolonne befindet, dadurch gekennzeichnet, dass die produktberührten Teile der Kondensationskolonne aus nichtrostendem Stahl sind, mindestens einer der Kondensationskolonne zugeführten Stoffströme eine Quelle für Halogenid-Ionen enthält und im Bereich der trennwirksamen Einbauten der Kondensationskolonne oberhalb des Seitenabzugs Halogenid-Ionen entfernt werden.

Der vorliegenden Erfindung liegt die Erkenntnis zugrunde, dass Halogenid-Ionen sich in der Kondensationskolonne oberhalb des Seitenabzugs anreichern können. Diese Halogenid-Ionen sind die Ursache für die unerwartete Korrosion. Wo die Konzentration der Halogenid-Ionen am höchsten ist, wird auch die höchste Korrosion festgestellt. Die Korrosion kann vermieden werden, wenn die Halogenid-Ionen gezielt entfernt werden.

Der C₃-Vorläufer der Acrylsäure ist vorzugsweise Propen und/oder Acrolein.

Der Halogenid-Ionen enthaltende Stoffstrom kann beispielsweise Wasser, Propen, Natronlauge, Hydrochinon, Hydrochinonmonomethylether, Diethylphthalat und/oder Phenothiazin sein. Die Halogenid-Ionen können in diesen oder anderen dem Verfahren zugeführten Stoffströmen als Verunreinigung enthalten sein.

Üblicherweise werden Fluorid-Ionen und Chlorid-Ionen als Halogenid-Ionen gefunden.

Die Kondensationskolonne enthält vorzugsweise Dual-Flow-Böden und Querstromböden als trennwirksamen Einbauten.

Die produktberührten Teile der Kondensationskolonne sind aus nichtrostendem Stahl. Nichtrostende Stähle im Sinne dieser Erfindung sind Stähle mit mindestens 10,5 Gew.-% Chrom.

Die bevorzugten nichtrostenden Stähle enthalten vorzugsweise16,0 bis 21,0 Gew.-%, besonders bevorzugt 17,0 bis 20,5 Gew.-%, ganz besonders bevorzugt 18,0 bis 20,0 Gew-%, Chrom und besonders bevorzugt zusätzlich vorzugsweise 8,0 bis 26,0 Gew.-%, besonders bevorzugt 10,0 bis 25,0 Gew.-%, ganz besonders bevorzugt 12,0 bis 24,0 Gew-%, Nickel und/oder zusätzlich vorzugsweise 2,0 bis 5,0 Gew.-%, besonders bevorzugt 2,5 bis 4,5 Gew.-%, ganz besonders bevorzugt 3,0 bis 4,0 Gew-%, Molybdän.

Weiterhin können die nichtrostenden Stähle vorteilhaft vorzugsweise 1,2 bis 2,0 Gew.-%, besonders bevorzugt 1,3 bis 1,9 Gew.-%, ganz besonders bevorzugt 1,4 bis 1,8 Gew-%, Kupfer enthalten.

In einer bevorzugten Ausführungsform der vorliegenden Erfindung wird eine Flüssigkeit F im Bereich der trennwirksamen Einbauten der Kondensationskolonne oberhalb des Seitenabzugs aus der Kondensationskolonne entnommen. Die Menge an entnommener Flüssigkeit F beträgt vorzugsweise von 0,0001 bis 0,5 Gew.-%, besonders bevorzugt von 0,001 bis 0,4 Gew.-%, ganz besonders bevorzugt von 0,01 bis 0,3 Gew.-%, jeweils bezogen auf die am Seitenabzug entnommene rohe Acrylsäure.

Aus der entnommenen Flüssigkeit F können die Halogenid-Ionen entfernt werden, beispielsweise Chlorid-Ionen mittels eines basischen Ionenaustauschers. Anschließend kann die von Halogenid-Ionen befreite Flüssigkeit F wieder in die Kondensationskolonne zurückgeführt werden.

Es ist aber auch möglich Halogenid-Ionen direkt in der Kondensationskolonne zu entfernen, beispielsweise Fluorid-Ionen durch Reaktion mit Glas, das zu diesem Zweck in die Kondensationskolonne eingebracht wurde.

Alternativ kann die entnommene Flüssigkeit F auch mit der aus der Kondensationskolonne ausgeschleusten Sumpfflüssigkeit vereinigt und mit diesem gemeinsam aufgearbeitet werden.

Alternativ kann die entnommene Flüssigkeit F auch mit dem aus der Kondensationskolonne ausgeschleusten sauren Wasser vereinigt und mit diesem gemeinsam aufgearbeitet werden.

Die Menge an Halogenid-Ionen, die aus der Kondensationskolonne entfernt werden, sollte so gewählt werden, dass der Halogenid-Gehalt in den Stoffströmen der Kondensationskolonne vorzugsweise weniger als 0,005 Gew.-%, besonders bevorzugt weniger als 0,002 Gew.-%, ganz besonders bevorzugt weniger als 0,001 Gew.-%, beträgt, jeweils bezogen auf den Stoffstrom. Dies bedeutet, dass die obengenannten Konzentrationen in jedem Teil der Kondensationskolonne unterschritten werden sollten.

Im Folgenden wird die Herstellung von Acrylsäure beschrieben:

In typischer Weise kann das Acrylsäure enthaltende Produktgasgemisch einer heterogen katalysierten Gasphasen-Partialoxidation von C₃-Vorläufern der Acrylsäure mit molekularem Sauerstoff an im festen Aggregatzustand befindlichen Katalysatoren z.B. folgende Gehalte (insbesondere dann, wenn als C₃-Vorläufer Propen verwendet wird) aufweisen:

| | |
|---|---|
| 1 bis 30 Gew.-% | Acrylsäure, |
| 0,05 bis 10 Gew.-% | molekularer Sauerstoff, |
| 1 bis 30 Gew.-% | Wasser, |
| > 0 bis 5 Gew.-% | Essigsäure, |
| > 0 bis 3 Gew.-% | Propionsäure, |
| > 0 bis 1 Gew.-% | Maleinsäure und/oder Maleinsäure-Anhydrid, |
| 0 bis 2 Gew.-% | Acrolein, |
| 0 bis 1 Gew.-% | Formaldehyd, |
| > 0 bis 1 Gew.-% | Furfurale, |
| > 0 bis 0,5 Gew.-% | Benzaldehyd, |
| 0 bis 1 Gew.-% | Propen, und als Restmenge im Wesentlichen inerte Gase wie z.B. Stickstoff, Kohlenmonoxid, Kohlendioxid, Methan und/oder Propan. |

Üblicherweise enthält das Produktgasgemisch, bezogen auf enthaltene Acrylsäure, ≥0,005 mol-%, häufig ≥0,03 mol-%, an Furfuralen. In der Regel beträgt der so bezogene Furfuralgehalt jedoch ≤3 mol.-%.

Die Gasphasen-Partialoxidation selbst kann wie im Stand der Technik beschrieben durchgeführt werden. Ausgehend von Propen kann die Gasphasen-Partialoxidation z.B. in zwei aufeinanderfolgenden Oxidationsstufen durchgeführt werden, wie sie in der EP 0 700 714 A1 und in der EP 0 700 893 A1 beschrieben sind. Selbstverständlich können aber auch die in der DE 197 40 253 A1 sowie in der DE 197 40 252 A1 zitierten Gasphasen-Partialoxidationen zur Anwendung kommen.

Im Sinne einer geringen Menge gebildeter Nebenkomponenten wird die Propen-Gasphasen-Partialoxidation bevorzugt wie in der DE 101 48 566 A1 beschrieben durchgeführt. Als Propen-Quelle kann dazu Polymer grade Propen oder Chemical grade Propen gemäß der DE 102 32 748 A1 verwendet werden. Handelt es sich bei dem verwendeten C₃-Vorläufer um Propan, kann die Partialoxidation wie in der DE 102 45 585 A1 beschrieben durchgeführt werden.

Grundsätzlich kann die Gasphasen-Partialoxidation aber auch wie in den Schriften US 2006/0161019, WO 2006/092410, WO 2006/002703, WO 2006/002713, WO 2005/113127, DE 10 2004 021 763 A1, EP 1 611 076 A1, WO 2005/108342, EP 1 656 335 A1, EP 1 682 478 A1, EP 1 682 477 A1, DE 10 2006 054 214 A1, DE 10 2006 024 901 A1, EP 1 611 080 A2, EP 1 734 030 A1, DE 10 2006 000 996 A1, DE 10 2005 062 026 A1, DE 10 2005 062 010 A1, WO 2007/060036, WO 2007/051750 sowie WO 2007/042457 beschrieben durchgeführt werden.

Häufig beträgt die Temperatur des die Gasphasen-Partialoxidation verlassenden Produktgasgemisches 150 bis 350°C, vielfach 200 bis 300°C, manchmal bis zu 500°C.

Anwendungstechnisch zweckmäßig wird das heiße Produktgasgemisch anschließend in einer Quenchvorrichtung 1 durch direkte Kühlung in der Regel auf eine Temperatur von 100 bis 180°C abgekühlt, bevor es, anwendungstechnisch vorteilhaft gemeinsam mit der verwendeten Quenchflüssigkeit 1, zum Zweck der fraktionierenden Kondensation bevorzugt in den unteren Abschnitt (bevorzugt den untersten, z.B. den Sumpfraum) einer trennwirksamen Einbauten enthaltenden Kondensationskolonne geleitet wird.

Als Kondensationskolonneneinbauten kommen prinzipiell alle gängigen Einbauten in Betracht, insbesondere Böden, Packungen und/oder Füllkörper. Von den Böden sind Glockenböden, Siebböden, Ventilböden und/oder Dual-Flow-Böden bevorzugt. In typischer Weise beträgt bei einer Bodenkolonne die Gesamtzahl an Trennböden 20 bis 100, häufig 20 bis 80 und bevorzugt 50 bis 80.

Erfindungsgemäß bevorzugt ist die Kondensationskolonne eine solche, die von unten nach oben, zunächst Dual-Flow-Böden und im Anschluss daran hydraulisch abgedichtete Querstromböden (z.B. Thormann-Böden) als trennwirksame Einbauten enthält, wie es die DE 102 43 625 A1, die DE 199 24 532 A1 und die DE 102 43 625 A1 empfehlen. Die Anzahl der Dual-Flow-Böden kann dabei 5 bis 60, häufig 25 bis 45, und die Anzahl der hydraulisch abgedichteten Querstromböden ebenfalls 5 bis 60, häufig 30 bis 50 betragen. Für den Bereich der Sauerwasserbildung (Acrylsäuregehalt der Rücklaufflüssigkeit von unten nach oben betrachtet in der Regel ≤15 Gew.-%, bzw. teilweise ≤10 Gew.-%) kommen als trennwirksame Einbauten bevorzugt Ventilböden in Betracht, wie es die DE 199 24 532 A1 und die DE 102 43 625 A1 beschreiben. Prinzipiell könnten aber auch andere gängige trennwirksame Einbauten verwendet werden (die einzelnen Bereiche innerhalb der Kondensationskolonne können natürlich in völlig äquivalenter Weise (anstelle in einer Kolonne übereinander) auch als eine Hintereinanderschaltung entsprechend kleinerer Kolonnen gestaltet werden).

Als Quenchvorrichtung 1 können alle im Stand der Technik für diesen Zweck bekannten Vorrichtungen (z.B. Sprühwäscher, Venturiwäscher, Blasensäulen oder sonstige Apparate mit berieselten Oberflächen) eingesetzt werden, wobei vorzugsweise Venturi-Wäscher oder Sprühkühler verwendet werden.

Zur indirekten Kühlung oder Erwärmung der Quenchflüssigkeit 1 wird diese, insbesondere beim Anfahren, bevorzugt, aber nicht in notwendiger Weise, über einen Wärmeüberträger bzw. Wärmeaustauscher geführt. Diesbezüglich eignen sich alle gängigen Wärmeüberträger oder Wärmeaustauscher. Als bevorzugt seien Rohrbündelwärmeaustauscher, Plattenwärmeaustauscher und Luftkühler genannt. Geeignete Kühlmedien sind Luft beim entsprechenden Luftkühler und Kühlflüssigkeiten, insbesondere Wasser, bei den anderen Kühlvorrichtungen.

Als Quenchflüssigkeit 1 kann z.B. aus dem Sumpf der Kondensationskolonne entnommene Sumpfflüssigkeit (gegebenenfalls vereinigt mit aus dem Quenchkreis 0 herausgeführtem Kondensat), oder über einen in Sumpfnähe gelegenen Seitenabzug Schwersiederfraktion oder ein Gemisch aus solcher Sumpfflüssigkeit und Schwersiederfraktion (insbesondere dann, wenn der Sumpfraum und der unterste (theoretische Boden (die unterste trennwirksame Einbaute) durch einen Kaminboden getrennt sind) verwendet werden. Gegebenenfalls wird nur der aus dem Sumpf der Kondensationskolonne entnommene Anteil der Quenchflüssigkeit 1 über den oben genannten Wärmeaustauscher geführt. Die Temperatur der Quenchflüssigkeit 1 beträgt beim Eintritt in die Quenchvorrichtung 1 in der Regel zweckmäßig 90°C bis 120°C.

Die Einleitstelle für das gequenchte (oder auf andere Art abgekühlte oder auch nicht abgekühlte) Produktgasgemisch der katalytischen Gasphasen-Partialoxidation (erfindungsgemäß wie beschrieben bevorzugt im Gemisch mit zur direkten Kühlung verwendeter Quenchflüssigkeit 1) in die Kondensationskolonne befindet sich vorteilhaft im Sumpfraum dieser Kolonne, der mit Vorteil einen Zentrifugaltropfenabscheider integriert enthält und in der Regel durch einen ersten Kaminboden von der untersten trennwirksamen Einbaute getrennt ist (anwendungstechnisch zweckmäßig leitet man in diesem Fall über eine Verbindungsleitung oder über einen Überlauf stetig Schwersiederfraktion in den Sumpf der Kondensationskolonne). In einer beispielhaften und bevorzugten Ausführungsvariante (die im Folgenden ohne Beschränkung der allgemeinen Ausführbarkeit ausschließlich beschrieben wird) handelt es sich bei dieser um den ersten Dual-Flow-Boden einer ersten Serie von zweckmäßig äquidistant angeordneten, Dual-Flow-Böden. Der Kaminboden fungiert gleichzeitig als Sammelboden, von dem kontinuierlich Kondensat (Schwersiederfraktion) entnommen und als Teil der Quenchflüssigkeit 1 in die Quenchvorrichtung 1 oder in den Sumpfraum geführt wird. Die erste Serie von Dual-Flow-Böden wird von einem zweiten Kaminboden (Fangboden) abgeschlossen. Von diesem zweiten Fangboden wird im ersten Seitenabzug als Mittelsiederfraktion rohe Acrylsäure kontinuierlich entnommen, die bevorzugt eine Reinheit von ≥90 Gew,-% bzw. ≥95 Gew.-% aufweist.

Zweckmäßigerweise wird man diese rohe Acrylsäure weiteren destillativen (rektifikativen) und/oder kristallisativen Weiterreinigungsstufen zuführen und wenigstens einen Teil der im Rahmen dieser Destillation (Rektifikation) und/oder Kristallisation anfallenden Sumpfflüssigkeiten und/oder Mutterlaugen unterhalb des ersten Seitenabzugs, aber oberhalb des ersten Fangbodens in die Kondensationskolonne rückführen. Vorzugsweise erfolgt diese Rückführung wärmeintegriert. D.h., kalte rückzuführende Mutterlauge wird durch einen oder mehrere hintereinander geschaltete indirekte Wärmeaustauscher (z.B. Spiralwärmeaustauscher) geführt, um in diesen die der Kondensationskolonne entnommene und im Wärmeaustauscher auf der gegenüberliegenden Seite geführte, kristallisativ weiter zu reinigende, rohe Acrylsäure abzukühlen. Gleichzeitig wird dadurch eine Erwärmung der Mutterlauge bewirkt. Vorzugsweise werden für diesen Zweck zwei hintereinandergeschaltete Plattenwärmeaustauscher verwendet.

In zweckmäßiger Weise wird man die (als Mittelsiederfraktion) entnommene rohe Acrylsäure zum Zweck der Weiterreinigung einer Kristallisation zuführen. Das zu verwendende Kristallisationsverfahren unterliegt prinzipiell keiner Beschränkung. Die Kristallisation kann kontinuierlich oder diskontinuierlich, einstufig oder mehrstufig bis zu beliebigen Reinheitsgraden durchgeführt werden.

Bei Bedarf kann der kristallisativ zu reinigenden rohen Acrylsäure vorab der Kristallisation vorteilhaft Wasser zugesetzt werden (in der Regel enthält diese dann, bezogen auf die enthaltene Menge an Acrylsäure, bis zu 20 Gew.-% oder bis zu 10 Gew.-%, meist bis zu 5 Gew.-% an Wasser). Bei erhöhten Aldehyd- oder sonstigen Nebenkomponentengehalten kann eine Wasserzugabe unterbleiben, da die Aldehyde in diesem Fall die Funktion des Wassers zu übernehmen vermögen. Erfindungsgemäß ganz besonders vorteilhaft wird das Wasser in Form von Sauerwasser zugesetzt. Dies führt zu einer Erhöhung der Ausbeute an Reinacrylsäure.

Es überrascht, dass selbst bei vorheriger Sauerwasserzugabe zur rohen Acrylsäure (diese Maßnahme bedingt gleichfalls eine Steigerung der Acrylsäureausbeute) eine höchsten Ansprüchen genügende veresterungsgerechte (z.B. für die Herstellung von n-Butylacrylat, 2-Ethylhexylacrylat, Methylacrylat und Ethylacrylat) Acrylsäure (Reinheit ≥98 Gew.-%) bereits durch eine einzige Kristallisationsstufe erzielt werden kann. Zweckmäßigerweise wird diese Kristallisationsstufe als Suspensionskristallisation ausgeführt, wie es in Spalte 10 der DE 199 24 532 A1 bzw. in Beispiel 1 der DE 102 23 058 A1 beschrieben ist (z.B. in einem Kühlscheibenkristallisator wie in der WO 2006/111565 beschrieben). Die bei der Suspensionskristallisation entstehenden Acrylsäure-Kristalle haben eine würfel- bis quaderförmige Erscheinungsform. Das Länge (L) zu Dicke (D) Verhältnis liegt dabei üblicherweise im Bereich von L:D = 1:1 bis L:D = 6:1, bevorzugt im Bereich 1:1 bis 4:1, und besonders bevorzugt im Bereich 1,5:1 bis 3,5:1. Die Dicke D der Kristalle liegt üblicherweise im Bereich von 20 bis 600 µm, oft bei 50 bis 300 µm. Die Länge L der Kristalle liegt üblicherweise im Bereich von 50 bis 1500 µm, oft bei 200 bis 800 µm. Die Abtrennung des Suspensionskristallisats von der verbliebenen Mutterlauge kann im Fall von veresterungsgerechter Acrylsäure auf einer Zentrifuge (z.B. einer 2- oder 3-stufigen Schubzentrifuge) erfolgen, wobei die abgetrennten Kristalle in vorteilhafter Weise auf der Zentrifuge mittels aufgeschmolzenem Reinkristallisat gewaschen werden. Trennt man das Suspensionskristallisat von der verbliebenen Mutterlauge mittels einer Waschkolonne, z.B. eine Schmelz-Waschkolonne (z.B. einer solchen gemäß der WO 01/77056, oder der DE 101 56 016 A1, oder der DE 102 23 058 A1, oder wie in der WO 2006/111565, der WO 04/35514, der WO 03/41833, der WO 02/09839, der WO 03/41832, der DE 100 36 881 A1, der WO 02/55469 sowie der WO 03/78378 beschrieben), kann mittels einer einzigen Kristallisationsstufe sogar superabsorbergerechte Acrylsäure (Reinheit ≥99,7 Gew.-% bzw. ≥99,9 Gew.-%), d.h., Acrylsäure, die sich zur Herstellung von Wasser superabsorbierenden oder sonstigen Polyacrylaten eignet, erzielt werden. In diesem Fall führt man in zweckmäßiger Weise die Gesamtmenge der abgetrennten Mutterlauge in die Kondensationskolonne zurück.

Die Kristallisation kann aber auch als fraktionierte Fallfilmkristallisation durchgeführt werden, wie sie die EP 0 616 998 A1 empfiehlt. Diese kann z.B. zwei, drei oder mehr (z.B. 2 bis 4) Reinigungsstufen umfassen (diesbezüglich geeignete Fallfilmkristallisatoren können z.B. 1000 bis 1400 Kristallisationsrohre einer Länge von 10 bis 15 m und eines Außendurchmessers von 50 bis 100 mm enthalten). Die in einer höheren Reinigungsstufe abgetrennte Mutterlauge kann in eine der vorhergehenden Reinigungsstufen rückgeführt werden. Die in der ersten Reinigungsstufe abgetrennte Mutterlauge wird vorteilhaft vollständig in die Kondensationskolonne rückgeführt. Alternativ zur Rückführung in eine der vorhergehenden Reinigungsstufen können die Mutterlaugen der einzelnen Reinigungsstufen auch in ihrer Gesamtmenge in die Kondensationskolonne rückgeführt werden. Das Reinprodukt der vorletzten Reinigungsstufe kann vollständig oder nur teilweise der letzten Reinigungsstufe zugeführt werden. Erfolgt nur eine Teilzuführung wird man die verbleibende Restmenge in der Regel mit dem Reinprodukt der letzten Reinigungsstufe zum dann verbrauchsgerechten Endprodukt abmischen.

Erfindungsgemäß zweckmäßig wird man eine Teilmenge der über den ersten Seitenabzug entnommenen rohen Acrylsäure dem unterhalb des zu diesem gehörigen Fangbodens befindlichen Dual-Flow-Boden zuführen. Diesem Boden wird man in der Regel auch gegebenenfalls in die Kondensationskolonne rückzuführende Mutterlauge zuführen. Vorab der Zufuhr wird man die Mutterlauge in der Regel, wie bereits beschrieben, wärmeintegriert auf eine Temperatur erwärmen, die in etwa der Entnahmetemperatur der rohen Acrylsäure entspricht.

Eine andere Teilmenge der über den ersten Seitenabzug entnommenen rohen Acrylsäure wird man vorteilhaft durch indirekten Wärmeaustausch um 10 bis 15°C erwärmen und oberhalb der Entnahmestelle, bevorzugt unmittelbar unterhalb des ersten nachfolgenden Dual-Flow-Bodens, in die Kondensationskolonne rückführen. Diese Maßnahme wirkt sich günstig auf den Essigsäuregehalt der entnommenen rohen Acrylsäure aus.

Oberhalb des zweiten Fangbodens schließt sich zunächst eine zweite Serie von, zweckmäßigerweise äquidistanten, Dual-Flow-Böden an, die dann von hydraulisch abgedichteten Querstrom-Stoffaustauschböden (z.B. Thormann-Böden oder modifizierte Thormann-Böden gemäß DE 102 43 625 A1), die zweckmäßigerweise gleichfalls äquidistant angeordnet sind, abgelöst werden. Der oberste Dual-Flow-Boden ist gegebenenfalls als Verteilerboden ausgerüstet. D.h., er weist z.B. Überlaufrinnen mit gezacktem Überlauf auf.

Der erste der Thormann-Böden von unten ist anwendungstechnisch zweckmäßig ein solcher, bei dem die vom Boden ablaufende Flüssigkeit über sechs als Rohre ausgebildete Ablaufschächte abläuft. Diese Rohre sind gegen den Gasraum des darunter liegenden Dual-Flow-Bodens hydraulisch abgedichtet. Die Wehrhöhen der sechs Ablaufrohre nehmen in Strömungsrichtung des Querstrombodens anwendungstechnisch zweckmäßig ab. Mit Vorteil weist die hydraulische Abdichtung Leerlauföffnungen mit Prallplatte auf. Die Ablaufrohre sind bevorzugt in der zweiten Hälfte, besonders bevorzugt im letzten Drittel des Bodenquerschnitts (dem Zulauf auf den Boden gegenüberliegend) gleichmäßig verteilt.

Die hydraulische Abdichtung erfolgt in eine Tasse mit schrägem Überlaufwehr (45°).

Die Querstrom-Stoffaustauschböden werden mit einem dritten Kaminboden (Fangboden) abgeschlossen.

Oberhalb des dritten Fangbodens befinden sich, vorzugsweise zweiflutige, Ventilböden. Das Prinzip von Ventilböden sowie erfindungsgemäß verwendbare Ventilböden finden sich z.B. in Technische Fortschrittsberichte, Band 61, Grundlagen der Dimensionierung von Kolonnenböden, Seiten 96 bis 138. Sie sind im Wesentlichen dadurch charakterisiert, dass sie dem durchströmenden Dampf über einen weiten Belastungsbereich eine der jeweiligen Belastung entsprechende Durchstromöffnung zur Verfügung stellen. Erfindungsgemäß bevorzugt werden Ballastböden verwendet. D.h., in den Öffnungen des Bodens befinden sich Käfige mit durch Gewichte verschlossenen Öffnungen. Erfindungsgemäß besonders bevorzugt sind VV12-Ventile der Fa. Stahl, Viernheim, Deutschland. Im Ventilbodenraum kondensiert im wesentlichen Wasser sowie schwerer als Wasser flüchtige Bestandteile. Das dabei gewonnene Kondensat ist Sauerwasser.

Vom dritten Fangboden wird im zweiten Seitenabzug kontinuierlich das Sauerwasser entnommen. Ein Teil des entnommenen Sauerwassers wird auf den obersten der Querstrom-Stoffaustauschböden in die Kondensationskolonne rückgeführt. Ein anderer Teil des entnommenen Sauerwassers wird durch indirekten Wärmetausch abgekühlt und, in zweckmäßiger Weise gesplittet, ebenfalls in die Kondensationskolonne rückgeführt. Eine gekühlte Teilmenge wird dabei auf den obersten Ventilboden (mit einer Temperatur von 15 bis 25, bevorzugt 20 bis 25°C) und die andere gekühlte Teilmenge auf einen zwischen dem dritten Fangboden und dem obersten Ventilboden etwa mittig gelegenen Ventilboden in die Kondensationskolonne rückgeführt (mit einer Temperatur von 20 bis 35, bevorzugt 25 bis 30°C). Aus der restlichen entnommenen Sauerwassermenge kann die enthaltene Acrylsäuremenge erfindungsgemäß abgetrennt werden.

Ein Teil der Abkühlung (die über einen oder mehrere hintereinandergeschaltete indirekte Wärmeaustauscher vorgenommen werden kann) wird dadurch bewirkt, dass die entsprechende Sauerwasserteilmenge über den Verdampfer des C₃-Vorläufers (z.B. den Propenverdampfer) geführt wird, um flüssig gelagerten C₃-Vorläufer, z.B. Propen, für die heterogen katalysierte Gasphasenoxidation in die Gasphase zu überführen.

Die leichter als Wasser flüchtigen Bestandteile werden am Kopf der Kondensationskolonne als Restgas (bzw. Restgasgemisch) gasförmig abgezogen und im Normalfall wenigstens teilweise als Verdünnungsgas (Kreisgas) in die Gasphasen-Partialoxidation rückgeführt. Um im Kreisgasverdichter Kondensation zu vermeiden, wird das Restgasgemisch zuvor durch indirekten Wärmeaustausch überhitzt. Der nicht im Kreis geführte Teil des Restgasgemisches wird normalerweise der Verbrennung zugeführt. Ein Teil des (vorzugsweise verdichteten) Restgasgemisches wird, wie bereits beschrieben, in vorteilhafter Weise als Strippgas zur Abtrennung von Acrylsäure aus dem Extrakt und aus der Sumpfflüssigkeit der Kondensationskolonne verwendet. Vorteilhaft wird die Gasphasen-Partialoxidation mit einem Überschuss an molekularem Sauerstoff durchgeführt, so dass das Restgasgemisch und damit das erste und das zweite Strippgas molekularen Sauerstoff enthalten, wenn Restgasgemisch als solches Strippgas verwendet wird.

Zum Zweck der Polymerisationsinhibierung wird dem obersten der hydraulisch abgedichteten Querstrom-Stoffaustauschböden eine Lösung von Hydrochinonmonomethylether (= MEHQ) in Acrylsäure oder (erfindungsgemäß bevorzugt) eine MEHQ-Schmelze und (in beiden Fällen) gegebenenfalls zusätzlich eine Lösung von Phenothiazin in Acrylsäure zugeführt. Als Acrylsäure wird dabei vorzugsweise eine reine Acrylsäure verwendet, wie sie bei der Weiterreinigung der entnommenen rohen Acrylsäure erzeugt wird. Beispielsweise kann die im Rahmen der kristallisativen Weiterreinigung erzeugte Reinacrylsäure (Reinprodukt) verwendet werden. Diese Lösung wird zweckmäßig auch zur Reinproduktstabilisierung verwendet.

Zusätzlich wird etwa in der Mitte des Kolonnenabschnitts mit den hydraulisch abgedichteten Querstrom-Stoffaustauschböden eine Lösung von Phenothiazin (= PTZ) in Reinprodukt zugeführt.

Prinzipiell kann die Sauerwasserbildung z.B. auch hinter einer ersten Kondensationskolonne praktiziert werden (vgl. DE 102 35 847 A1). In diesem Fall wird man aus dem dann am Kopf der ersten Kondensationskolonne entweichenden Leichtsiedergasstrom in zweckmäßiger Weise durch direkte Kühlung in einem an Einbauten freien oder Einbauten enthaltenden nachgeschalteten Raum (zweite Kolonne) mittels einer Quenchflüssigkeit 2 im wesentlichen Wasser auskondensieren. Das dabei gewonnene Kondensat ist wiederum das Sauerwasser. Einen Teil des Sauerwassers wird man dann in sinnvoller Weise zur Erhöhung der Trennleistung am Kopf der ersten Kondensationskolonne in selbige rückführen. Ein weiterer Teil des Sauerwassers wird in einem externen Wärmetauscher indirekt abgekühlt und als die Quenchflüssigkeit 2 verwendet und aus der restlichen Sauerwassermenge kann die Acrylsäure wiederum erfindungsgemäß extrahiert werden. Leichter als Wasser flüchtige Bestandteile des Leichtsiederstroms bilden wiederum Restgas, das normalerweise wenigstens teilweise als Kreisgas in die Gasphasen-Partialoxidation rückgeführt bzw. als Strippgas verwendet wird.

Zweckmäßig erstrecken sich die Dual-Flow-Böden bei der bevorzugten Variante des erfindungsgemäßen Verfahrens in der Kondensationskolonne in etwa bis zu dem Querschnitt in der Kondensationskolonne, von dem ab die Acrylsäuregehalte der Rücklaufflüssigkeit zum Kolonnenkopf hin betrachtet ≤90 Gew.-%, bezogen auf das Gewicht der Rücklaufflüssigkeit, betragen.

Die Anzahl an Dual-Flow-Böden beträgt, wie bereits gesagt, für die beschriebene Vorzugsvariante der fraktionierenden Kondensation in der Regel 25 bis 45. Ihr Öffnungsverhältnis liegt zweckmäßig bei 12 bis 25%. Als Durchtrittsstellen weisen die Dual-Flow-Böden dabei bevorzugt kreisrunde Löcher mit einem einheitlichen Kreisdurchmesser auf. Letzterer beträgt zweckmäßig 10 bis 20 mm. Bei Bedarf kann man in der Kondensationskolonne die Lochdurchmesser von oben nach unten verjüngen oder vergrößern und/oder die Anzahl der Löcher vermindern oder vergrößern (z.B. kann der Lochdurchmesser einheitlich 14 mm betragen und das Öffnungsverhältnis von oben nach unten von 17,4% auf 18,3% zunehmen). Jedoch kann die Lochanzahl auch über alle Dual-Flow-Böden konstant sein. Ferner sind die kreisrunden Löcher über den individuellen Dual-Flow-Böden bevorzugt in strenger Dreiecksteilung gleichmäßig angeordnet (vgl. DE 102 30 219 A1).

Außerdem zeigt der Stanzgrat der in den Dual-Flow-Böden herausgestanzten Durchtrittsöffnungen in der Kondensationskolonne bevorzugt nach unten (unerwünschte Polymerbildung wird dadurch gemindert).

Erfindungsgemäß sinnvoll ist es, wenn die Anzahl der in der Kondensationskolonne eingesetzten Dual-Flow-Böden etwa 10 bis 15 theoretischen Trennstufen entspricht.

Die Anzahl der sich an die Dual-Flow-Böden in der erfindungsgemäß bevorzugten Kondensationskolonne anschließenden hydraulisch abgedichteten Querstrom-Stoffaustauschböden wird, wie bereits erwähnt, in der Regel 30 bis 50 betragen. Ihr Öffnungsverhältnis wird zweckmäßig 5 bis 25%, bevorzugt 10 bis 20% betragen (das Öffnungsverhältnis gibt ganz generell den prozentualen Anteil der Durchtrittsquerschnitte am Gesamtquerschnitt wieder; es liegt bei den vorzugsweise mitzuverwendenden Querstrom-Stoffaustauschböden ganz generell zweckmäßig im vorgenannten Bereich).

Einflutige Querstrom-Stoffaustauschböden werden erfindungsgemäß bevorzugt eingesetzt.

In der Regel wird die Anzahl der hydraulisch abgedichteten Querstromböden für die Vorzugsvariante der fraktionierenden Produktgasgemischkondensation so bemessen, dass sie etwa 10 bis 30, häufig 25 theoretischen Trennstufen entspricht.

Sowohl die hydraulisch abgedichteten Querstromböden als auch gegebenenfalls mitverwendete Ventilböden weisen wenigstens einen Ablaufschacht auf. Sie können beide sowohl einflutig als auch mehrflutig, z.B. zweiflutig gestaltet sein. Dabei können sie auch bei einflutiger Gestaltung mehr als einen Ablaufschacht aufweisen. In der Regel sind auch die Zulaufschächte der Ventilböden hydraulisch abgedichtet.

Die Polymerisationsinhibierung des Quenchsystems 1 für das Produktgasgemisch der partiellen Gasphasenoxidation kann sowohl über zum Quenchen verwendeter Sumpfflüssigkeit (aus der Kondensationskolonne) enthaltene Polymerisationsinhibitoren als auch über zum Quenchen verwendeter Schwersiederfraktion (aus der Kondensationskolonne) enthaltene Polymerisationsinhibitoren bewerkstelligt werden.

Der Vorteil des erfindungsgemäßen Verfahrens liegt nochmals darin begründet, dass es bei im Wesentlichen gleicher Reinheit eine erhöhte Ausbeute an roher Acrylsäure ermöglicht. Alle in dieser Schrift gemachten Aussagen gelten insbesondere für ein Produktgasgemisch, das durch (vorzugsweise zweistufige) heterogene Partialoxidation von Propen zu Acrylsäure erhalten wurde. Die vorstehend beschriebene bevorzugte Ausführungsvariante des erfindungsgemäßen Verfahrens beschränkt in keiner Weise die allgemeine Ausführbarkeit.

Abschließend sei noch festgehalten, dass sowohl das erste Strippgas als auch das zweite Strippgas mit Vorteil molekularen Sauerstoff enthält.

### Beispiele

### Beispiel 1 (Vergleichsbeispiel)

Es wurde verfahren wie in Beispiel 1 der EP 2 114 852 A1. Eingetragenes Chlorid reicherte sich im Bereich der Thormann-Böden an. Der Chlorid-Gehalt betrug bis zu 115 ppm. Die Thormann-Böden waren aus nichtrostendem Stahl (Werkstoff 1.4571 gemäß DIN EN 10088: 16,5 bis 18,5 Gew.-% Chrom, 10,5 bis 13,5 Gew.-% Nickel, 2,0 bis 2,5 Gew.-% Molybdän, bis zu 0,7 Gew.-% Titan). An den Thormann-Böden 5 bis 7 (vom untersten Thormann-Boden gezählt) wurde Korrosion beobachtet.

### Beispiel 2

Es wird verfahren wie in Beispiel 1. Von Thormann-Boden 6 (vom untersten Thormann-Boden gezählt) werden 80 bis 205 kg/h Flüssigkeit abgezogen. Die Flüssigkeitsmenge wird so geregelt, dass der Chlorid-Gehalt unter 10 ppm bleibt. Die ausgeschleuste Flüssigkeit kann in die Sauerwasserextraktion oder die zweite Strippkolonne rückgeführt werden.

## Patentansprüche

1. Verfahren zur Herstellung von Acrylsäure, bei dem man durch heterogen katalysierte Gasphasen-Partialoxidation wenigstens eines C₃-Vorläufers der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt, das Produktgasgemisch anschließend in eine mit trennwirksamen Einbauten ausgerüstete Kondensationskolonne leitet, das Produktgasgemisch innerhalb der Kondensationskolonne in sich selbst aufsteigen lässt und dabei fraktionierend kondensiert, wobei das Produktgasgemisch in eine schwerer als Acrylsäure siedende Folgeprodukte und Nebenkomponenten enthaltende Sumpfflüssigkeit, eine Wasser und Nebenkomponenten insgesamt entreichert enthaltende rohe Acrylsäure als Zielprodukt, ein noch Acrylsäure und Nebenkomponenten enthaltendes saures Wasser und ein tiefer als Wasser siedende Nebenkomponenten enthaltendes Restgasgemisch aufgetrennt wird, das Zielprodukt über einen Seitenabzug aus der Kondensationskolonne herausführt und sich der Seitenabzug oberhalb der Zuführstelle des Produktgasgemischs in die Kondensationskolonne befindet, **dadurch gekennzeichnet, dass** die produktberührten Teile der Kondensationskolonne aus nichtrostendem Stahl sind, mindestens einer der Kondensationskolonne zugeführten Stoffströme eine Quelle für Halogenid-Ionen enthält und im Bereich der trennwirksamen Einbauten der Kondensationskolonne oberhalb des Seitenabzugs Halogenid-Ionen entfernt werden.

2. Verfahren nach Anspruch 1, **dadurch gekennzeichnet, dass** die Halogenid-Ionen Fluorid-Ionen und/oder Chlorid-Ionen sind.

3. Verfahren nach Anspruch 1 oder 2, **dadurch gekennzeichnet, dass** der C₃-Vorläufer der Acrylsäure Propen und/oder Acrolein ist.

4. Verfahren nach einem der Ansprüche 1 bis 3, **dadurch gekennzeichnet, dass** der eine Quelle für Halogenid-Ionen enthaltende Stoffstrom Wasser, Propen, Natronlauge, Hydrochinon, Hydrochinonmonomethylether, Diethylphthalat und/oder Phenothiazin ist.

5. Verfahren nach einem der Ansprüche 1 bis 4, **dadurch gekennzeichnet, dass** Dual-Flow-Böden und Querstromböden als trennwirksamen Einbauten der Kondensationskolonne verwendet werden.

6. Verfahren nach einem der Ansprüche 1 bis 5, **dadurch gekennzeichnet, dass** die produktberührten Teile der Kondensationskolonne aus nichtrostendem Stahl mit 16 bis 21 Gew.-% Chrom und 8 bis 26 Gew.-% Nickel sind.

7. Verfahren nach Anspruch 6, **dadurch gekennzeichnet, dass** die produktberührten Teile der Kondensationskolonne aus nichtrostendem Stahl mit zusätzlich 2 bis 5 Gew.-% Molybdän sind.

8. Verfahren nach Anspruch 7, **dadurch gekennzeichnet, dass** die produktberührten Teile der Kondensationskolonne aus nichtrostendem Stahl mit zusätzlich 1,2 bis 2,0 Gew.-% Kupfer sind.

9. Verfahren nach einem der Ansprüche 1 bis 8, **dadurch gekennzeichnet, dass** eine Flüssigkeit F im Bereich der trennwirksamen Einbauten der Kondensationskolonne oberhalb des Seitenabzugs aus der Kondensationskolonne entnommen wird.

10. Verfahren nach Anspruch 9, **dadurch gekennzeichnet, dass** von 0,0001 bis 0,5 Gew.-% Flüssigkeit F entnommen werden, bezogen auf die am Seitenabzug entnommene rohe Acrylsäure.

11. Verfahren nach Anspruch 9 oder 10, **dadurch gekennzeichnet, dass** Halogenid-Ionen aus der Flüssigkeit F entfernt werden und die Flüssigkeit F anschließend in die Kondensationskolonne zurückgeführt wird.

12. Verfahren nach Anspruch 11, **dadurch gekennzeichnet, dass** die Halogenid-Ionen mittels eines basischen Ionenaustauschers aus der Flüssigkeit F entfernt werden.

13. Verfahren nach Anspruch 9 oder 10, **dadurch gekennzeichnet, dass** die Flüssigkeit F mit der aus der Kondensationskolonne ausgeschleusten Sumpfflüssigkeit vereinigt wird.

14. Verfahren nach Anspruch 9 oder 10, **dadurch gekennzeichnet, dass** die Flüssigkeit F mit dem aus der Kondensationskolonne ausgeschleusten sauren Wasser vereinigt wird.

15. Verfahren nach einem der Ansprüche 1 bis 14, **dadurch gekennzeichnet, dass** das der Halogenid-Gehalt in den Stoffströmen der Kondensationskolonne weniger als 0,002 Gew.-% beträgt, bezogen auf den Stoffstrom.

## Claims

1. A process for preparing acrylic acid, in which heterogeneously catalyzed gas phase partial oxidation of at least one C₃ precursor of acrylic acid with molecular oxygen over catalysts in the solid state of matter at elevated temperature affords a product gas mixture comprising acrylic acid, water vapor and secondary components, then the product gas mixture is directed into a condensation column equipped with separating internals, the product gas mixture is allowed to ascend into itself within the condensation column and undergoes fractional condensation, separating the product gas mixture into a bottoms liquid comprising conversion products and secondary components that are higher-boiling than acrylic acid, a crude acrylic acid comprising water and secondary components that have been depleted overall as target product, an acid water still comprising acrylic acid and secondary components, and a residual gas mixture comprising secondary components that are lower-boiling than water, the target product is conducted out of the condensation column via a side draw and the side draw is above the feed point of the product gas mixture into the condensation column, wherein the parts of the condensation column that are in contact with product are made of stainless steel, at least one of the streams of matter fed to the condensation column comprises a source for halide ions, and halide ions are removed in the region of the separating internals of the condensation column above the side draw.

2. The process according to claim 1, wherein the halide ions are fluoride ions and/or chloride ions.

3. The process according to claim 1 or 2, wherein the C₃ precursor of acrylic acid is propene and/or acrolein.

4. The process according to any of claims 1 to 3, wherein the stream of matter comprising a source for halide ions is water, propene, sodium hydroxide solution, hydroquinone, hydroquinone monomethyl ether, diethyl phthalate and/or phenothiazine.

5. The process according to any of claims 1 to 4, wherein dual-flow trays and crossflow trays are used as separating internals in the condensation column.

6. The process according to any of claims 1 to 5, wherein the parts of the condensation column that are in contact with product are made from stainless steel having 16% to 21% by weight of chromium and 8% to 26% by weight of nickel.

7. The process according to claim 6, wherein the parts of the condensation column that are in contact with product are made from stainless steel additionally having 2% to 5% by weight of molybdenum.

8. The process according to claim 7, wherein the parts of the condensation column that are in contact with product are made from stainless steel additionally having 1.2% to 2.0% by weight of copper.

9. The process according to any of claims 1 to 8, wherein a liquid F is withdrawn from the condensation column in the region of the separating internals of the condensation column above the side draw.

10. The process according to claim 9, wherein from 0.0001% to 0.5% by weight of liquid F is withdrawn, based on the crude acrylic acid withdrawn in the side draw.

11. The process according to claim 9 or 10, wherein halide ions are removed from the liquid F, and then the liquid F is recycled into the condensation column.

12. The process according to claim 11, wherein the halide ions are removed from the liquid F by means of a basic ion exchanger.

13. The process according to claim 9 or 10, wherein the liquid F is combined with the bottoms liquid discharged from the condensation column.

14. The process according to claim 9 or 10, wherein the liquid F is combined with the acid water discharged from the condensation column.

15. The process according to any of claims 1 to 14, wherein the halide content in the streams of matter from the condensation column is less than 0.002% by weight, based on the stream of matter.

## Revendications

1. Procédé de production d'acide acrylique, dans lequel on produit à température élevée un mélange gazeux produit contenant de l'acide acrylique, de la vapeur d'eau et des composants secondaires par oxydation partielle en phase gazeuse à catalyse hétérogène d'au moins un précurseur en C₃ de l'acide acrylique avec de l'oxygène moléculaire sur des catalyseurs se trouvant à l'état d'agrégat solide, le mélange gazeux produit est ensuite conduit dans une colonne de condensation munie d'inserts à fonction de séparation, le mélange gazeux produit est laissé s'élever spontanément à l'intérieur de la colonne de condensation et y est condensé de façon fractionnée, le mélange gazeux produit étant fractionné en un liquide de pied de colonne contenant des produits dérivés et composants secondaires à plus haut point d'ébullition que l'acide acrylique, un acide acrylique brut en tant que produit cible, contenant de l'eau et globalement appauvri en composants secondaires, une eau acide contenant encore de l'acide acrylique et des composants secondaires et un mélange gazeux résiduel contenant des composants secondaires à plus bas point d'ébullition que l'eau, le produit cible est évacué de la colonne de condensation via un conduit de décharge latéral et le conduit de décharge latéral se trouve dans la colonne de condensation au-dessus du point d'introduction du mélange gazeux produit, **caractérisé en ce que** les parties de la colonne de condensation qui sont en contact avec le produit sont en acier inoxydable, au moins un des courants de substances envoyés à la colonne de condensation contient une source d'ions halogénure et des ions halogénure sont éliminés dans la zone des inserts à fonction de séparation de la colonne de condensation au-dessus du conduit de décharge latéral.

2. Procédé selon la revendication 1, **caractérisé en ce que** les ions halogénure sont des ions fluorure et/ou des ions chlorure.

3. Procédé selon la revendication 1 ou 2, **caractérisé en ce que** le précurseur en C₃ de l'acide acrylique est le propène et/ou l'acroléine.

4. Procédé selon l'une quelconque des revendications 1 à 3, **caractérisé en ce que** le courant de substances contenant une source d'ions halogénure est de l'eau, du propène, une solution d'hydroxyde de sodium, de l'hydroquinone, de l'éther monométhylique d'hydroquinone, du phtalate de diéthyle et/ou de la phénothiazine.

5. Procédé selon l'une quelconque des revendications 1 à 4, **caractérisé en ce que** des plateaux à double flux et des plateaux à écoulement transversal sont utilisés en tant qu'inserts à fonction de séparation de la colonne de condensation.

6. Procédé selon l'une quelconque des revendications 1 à 5, **caractérisé en ce que** les parties de la colonne de condensation qui sont en contact avec le produit sont en acier inoxydable comportant 16 à 21 % en poids de chrome et 8 à 26 % en poids de nickel.

7. Procédé selon la revendication 6, **caractérisé en ce que** les parties de la colonne de condensation qui sont en contact avec le produit sont en acier inoxydable comportant en plus 2 à 5 % en poids de molybdène.

8. Procédé selon la revendication 7, **caractérisé en ce que** les parties de la colonne de condensation qui sont en contact avec le produit sont en acier inoxydable comportant en plus 1,2 à 2,0 % en poids de cuivre.

9. Procédé selon l'une quelconque des revendications 1 à 8, **caractérisé en ce qu'**un liquide F est prélevé de la colonne de condensation dans la zone des inserts à fonction de séparation de la colonne de condensation au-dessus du conduit de décharge latéral.

10. Procédé selon la revendication 9, **caractérisé en ce que** de 0,0001 à 0,5 % en poids de liquide F sont prélevés, par rapport à l'acide acrylique brut prélevé au niveau du conduit de décharge latéral.

11. Procédé selon la revendication 9 ou 10, **caractérisé en ce que** des ions halogénure sont éliminés du liquide F et le liquide F est ensuite renvoyé dans la colonne de condensation.

12. Procédé selon la revendication 11, **caractérisé en ce que** les ions halogénure sont éliminés du liquide F au moyen d'un échangeur d'ions basique.

13. Procédé selon la revendication 9 ou 10, **caractérisé en ce que** le liquide F est réuni avec le liquide de pied de colonne évacué de la colonne de condensation.

14. Procédé selon la revendication 9 ou 10, **caractérisé en ce que** le liquide F est réuni avec l'eau acide évacuée de la colonne de condensation.

15. Procédé selon l'une quelconque des revendications 1 à 14, **caractérisé en ce que** la teneur en halogénures des courants de substances de la colonne de condensation est inférieure à 0,002 % en poids, par rapport au courant de substances.
